Europäisches Patentamt

European Patent Office

Office européen des brevets

(11) Publication number: **0 384 410 B1**

(12) **EUROPEAN PATENT SPECIFICATION**

(45) Date of publication of patent specification: **17.05.95**

(51) Int. Cl.⁶: **A61K 31/545**, A61K 31/43, //(A61K31/545,31:43)

(21) Application number: **90103266.4**

(22) Date of filing: **20.02.90**

The file contains technical information submitted after the application was filed and not included in this specification

(54) **Antimicrobial composition.**

(30) Priority: **21.02.89 JP 41286/89**
**14.04.89 JP 94460/89**

(43) Date of publication of application:
**29.08.90 Bulletin 90/35**

(45) Publication of the grant of the patent:
**17.05.95 Bulletin 95/20**

(84) Designated Contracting States:
**CH DE FR GB IT LI NL**

(56) References cited:
**EP-A- 0 248 361**

**UNLISTED DRUGS, vol. 37, no. 2, February 1985, Chatham, New Jersey, US; "Zienam 250".**

(73) Proprietor: **BANYU PHARMACEUTICAL CO., LTD.**
**2-3, Nihonbashi Honcho 2-chome**
**Chuo-ku, Tokyo (JP)**

(72) Inventor: **Matsuda, Kouji, c/o BANYU PHARM. CO., LTD.**
**OKAZAKI RES. LABORATORY,**

**9-1, Kamimutsuna 3-chome**
**Okazaki-shi,**
**Aichi (JP)**
Inventor: **Sanada, Minoru, c/o BANYU PHARM. CO., LTD.**
**OKAZAKI RES. LABORATORY,**
**9-1, Kamimutsuna 3-chome**
**Okazaki-shi,**
**Aichi (JP)**
Inventor: **Nakagawa, Susumu, c/o BANYU PHARM. CO., LTD.**
**OKAZAKI RES. LABORATORY,**
**9-1, Kamimutsuna 3-chome**
**Okazaki-shi,**
**Aichi (JP)**
Inventor: **Tanaka, Nobuo, c/o BANYU PHARM. CO., LTD.**
**2-3, Nihonbashi Honcho 2-chome**
**Chuo-ku,**
**Tokyo (JP)**
Inventor: **Inoue, Matsuhisa**
**3076-3, Oaza-Tokisawa**
**Fujimi-mura,**
**Seta-gun,**
**Gunma (JP)**

Rank Xerox (UK) Business Services
(3.10/3.09/3.3.3)

EP 0 384 410 B1

(74) Representative: **Klunker . Schmitt-Nilson .
Hirsch
Winzererstrasse 106
D-80797 München (DE)**

**Description**

BACKGROUND OF THE INVENTION

1. Field of the Invention

The present invention relates to a combination of different kinds of $\beta$-lactam antibiotics as a pharmaceutical composition, to the use of the composition for producing a medicament, and to products containing the combination.

2. Description of the Related Art

Currently, although many kinds of antibiotics have been used for the therapy of various infectious diseases, due to a tendency of pathogenic microorganisms which have encountered antibiotics to acquire a resistance to the antibiotics, usually microorganisms exist which are resistant to a single antibiotic. To resolve this dilemma, attempts have been made to use a combination of more than one antibiotic.

For example, the use of a combination of more than one antibiotic has been proposed to enhance an antimicrobial activity against pathogenic microorganisms, for example, gram-positive bacteria such as Staphyrococcus aureus, S. epidermidis, Enterococcus faecalis, etc.; gram negative bacteria such as Citrobacter freundii, Escherichia coli, Klebsiella pneumoniae, Proteus vulgaris, Serratia marcescens, etc.; and glucose non-fermentative gram-negative rods, such as Pseudomonas aeruginosa, P. cepacia, P. maltophilia, Acinetobacter calcoaceticus, etc.

Japanese Unexamined Patent Publication (KOKAI) No. 60-126230 and Japanese Examined Patent Publication (KOKOKU) No. 61-37248 describe antimicrobial compositions comprising a combination of penicillin series antibiotic, and penicillin or cephalosporin series antibiotic; Japanese Examined Patent Publication (KOKOKU) No. 62-50448 describes antimicrobial compositions comprising a combination of a synthetic antimicrobial substance and penicillin or cephalosporin series antibiotic; and Japanese Examined Patent Publication (KOKOKU) No. 63-26732 describes antimicrobial compositions comprising a combination of a phosphoric derivative and one of various antimicrobial substances.

From EP-A-0 248 361 it is known that combinations of certain cephalosporins and penems exhibit a synergistic effect and may be used for treating bacterial infections.

There is a tendency toward an increase in drug-resistant microorganisms isolated from patients suffering from intractable diseases, and there are few antimicrobial compositions which exhibit an effective and sufficient antimicrobial activity against methicillin resistant Staphylococcus aureus (MRSA), a representative pathogen of such diseases, and this has led to serious clinical difficulties.

It is well known that carbapenem series compounds have a wide antimicrobial spectrum. Known carbapenem series compounds include imipenem (Japanese Examined Patent Publication No. 61-60816), SM-7338 (Japanese Unexamined Patent Publication No. 60-10488), RS-533 (Japanese Unexamined Patent Publication No. 59-51286), but the administering of this carbapenem alone does not provide a sufficient antimicrobial activity against MRSA. Further, although imipenem is often used for the therapy of MRSA infections, it does not provide satisfactory results as MRSA is highly resistant to $\beta$-lactams.

All of the above references refer to tests on usual gram-positive bacteria, gram negative bacteria and glucose non-fermetntative gram-negative rods, and do not refer to an evaluation of an acitivity against MRSA.

Since there are limitations to the therapy of MRSA infections by a single agent, the use of a combination of an aminoglycoside and a $\beta$-lactam, or a combination of phosphomycin and a $\beta$-lactam has been attempted, but these combinations are not satisfactorily effective.

Since an infection with MRSA is representative of intractable infections, the deficiency in effective therapeutic agents must be urgently resolved.

SUMMARY OF THE INVENTION

Accordingly, the present invention provides an antimicrobial composition comprising a combination of (1) a first ingredient selected from penicillin series compounds, cephalosporin series compounds and pharmaceutically acceptable salts thereof; and (2) a second ingredient selected from carbapenem series compounds and pharmaceutically acceptable salts thereof wherein the penicillin series compound is selected from penicillin G, ampicillin, amoxicillin, mecillinam, pivmecillinam, carbenicillin, carfecillin, carindacillin, sulbenicillin, talampicillin, bacampicillin, ticarcillin, piperacillin, ciclacillin and hetacillin, the

3

cephalosporin series compound is selected from cefatrizine, cefamandole, cefuzoname, cefpimizole, cephapirin, cephaloridine, cefsulodin, cefotiam, ceforanide, ceftexzole, cefoxitin, latamoxef, flomoxef, cefmetazole, cefotetan, cefpiramide, cephaloglycin, cephalexin, cefadroxil, cefroxadine, ceferadine, cefaclor and cefoperazone, the carbapenem series compound is selected from imipenem, i.e., (5R,6S,8R)-3-[{2-(formimidoylamino)ethyl}thio] -6-(1-hydroxyethyl)-7-oxo-1-azabicyclo[3,2,0]hept-2-ene-2-carboxylic acid monohydrate, SM-7338, i.e. (4R,5S,6S,8R,2'S,4'S)-3-[2-(dimethylaminocarbonyl)pyrrolidin-4-ylthio]-6-(1-hydroxy-ethyl)-4-methyl-7-oxo-1-azabicyclo[3,2,0]hept-2-ene-2-carboxylic acid, and RS-533,i.e. (5R,6S, 8R,4'S)-3-[1-acetimidoylpyrrolidin-4-ylthio]-6-(1-hydroxyethyl)-7-oxo-1-azabicyclo[3,2,0]-hept-2-ene-2-carboxylic acid; and said combination providing a synergistic effect over the compounds used alone.

The composition of the present invention is effective against MRSA which is highly resistant to $\beta$-lactam antibiotics.

Further developments of the antimicrobial composition of the present invention result from the dependent claims 2 to 10.

The present invention also provides products containing a first antibiotic and a second antibiotic, wherein the first antibiotic is selected from penicillin series compounds, cephalosporin series compounds and pharmaceutically acceptable salts thereof and the second antibiotic is selected from carbapenem series compounds and pharmaceutically acceptable salts thereof, and wherein the penicillin series compound is selected from penicillin G, ampicillin, amoxicillin, mecillinam, pivmecillinam, carbenicillin, carfecillin, carindacillin, sulbenicillin, talampicillin, bacampicillin, ticarcillin, piperacillin, ciclacillin and hetacillin, the cephalosporin series compound is selected from cefatrizine, cefamandole, cefuzoname, cefpimizole, cephapirin, cephaloridine, cefsulodin, cefotiam, ceforanide, ceftexzole, cefoxitin, latamoxef, flomoxef, cefmetazole, cefotetan, cefpiramide, cephaloglycin, cephalexin, cefadroxil, cefroxadine, ceferadine, cefaclor and cefoperazone, the carbapenem series compound is selected from imipenem, i.e., (5R,6S,8R)-3-[{2-(formimidoylamino)ethyl}thio] -6-(1-hydroxyethyl)-7-oxo-1-azabicyclo[3,2,0]hept-2-ene-2-carboxylic acid monohydrate, SM-7338, i.e. (4R,5S,6S,8R,2'S,4'S)-3-[2-(dimethylaminocarbonyl)-pyrrolidin-4-ylthio]-6-(1-hydroxy-ethyl)-4-methyl-7-oxo-1-azabicyclo 3,2,0]hept-2-ene-2-carboxylic acid, and RS-533,i.e. (5R,6S, 8R,4'S)-3-[1-acetimidoylpyrrolidin-4-ylthio]-6-(1-hydroxyethyl)-7-oxo-1-azabicyclo[3,2,0]-hept-2-ene-2-carboxylic acid; and said combination providing a synergistic effect over the compounds used alone, the product containing the first antibiotic and the second antibiotic as a combined preparation for separate use or separately administering in the MRSA infection therapy.

The present invention further relates to the use of an antimicrobial composition comprising a combination of (1) a first ingredient selected from penicillin series compounds, cephalosporin series compounds and pharmaceutically acceptable salts thereof; and (2) a second ingredient selected from carbapenem series compounds and pharmaceutically acceptable salts thereof wherein the penicillin series compound is selected from penicillin G, ampicillin, amoxicillin, mecillinam, pivmecillinam, carbenicillin, carfecillin, carindacillin, sulbenicillin, talampicillin, bacampicillin, ticarcillin, piperacillin, ciclacillin and hetacillin, the cephalosporin series compound is selected from cefatrizine, cefamandole, cefuzoname, cefpimizole, cephapirin, cephaloridine, cefsulodin, cefotiam, ceforanide, ceftexzole, cefoxitin, latamoxef, flomoxef, cefmetazole, cefotetan, cefpiramide, cephaloglycin, cephalexin, cefadroxil, cefroxadine, ceferadine, cefaclor and cefoperazone, the carbapenem series compound is selected from imipenem, i.e., (5R,6S,8R)-3-[{2-(formimidoylamino)ethyl}thio]-6-(1-hydroxyethyl)-7-oxo-1-azabicyclo[3,2,0]hept-2-ene-2-carboxylic acid monohydrate, SM-7338, i.e.; (4R,5S,6S,8R,2'S, 4'S)-3-[2-(dimethylaminocarbonyl)pyrrolidin-4-ylthio]-6-(1-hydroxy-ethyl)-4-methyl-7-oxo-1-azabicyclo[3,2,0] hept-2-ene-2-carboxylic acid, and RS-533, i.e. (5R,6S,8R,4'S)-3-[1-acetimidoylpyrrolidin-4-ylthio] -6-(1-hydroxyethyl)-7-oxo-1-azabicyclo[3,2,0]-hept-2-ene-2-carboxylic acid; and said combination providing a syngergistic effect over the compounds used alone for producing a medicament for treating MRSA infections.

DESCRIPTION OF THE PREFERRED EMBODIMENTS

The penicillin series compounds and cephalosporin series compounds which are used in the present invention are antibiotics as listed below which, in combination with carbapenem series compounds as listed below or pharmaceutically acceptable salts thereof, provide a synergistic antimicrobial activity. Such antibiotics are known penicillin series compounds and known cephalosporin series compounds, namely the penicillin series compounds penicillin G, ampicillin, amoxicillin, mecillinam, pivmecillinam, carbenicillin, carfecillin, carindacillin, sulbenicillin, talampicillin, bacampicillin, ticarcillin, piperacillin, ciclacillin, hetacillin. In the present invention, ampicillin and piperacillin are preferable, and piperacillin is especially preferable.

The cephalosporin series compounds, are cefatrizine, cefamandole, cefuzoname, cefpimizole, cephapirin, cephaloridine, cefsulodin, cefotiam, ceforanide, ceftezole, cefoxitin, latamoxef, flomoxef, cefmetazole, cefotetan, cefpiramide, cephaloglycin, cephalexin, cefadroxil, cefroxadine, cefradine, cefaclor, cefoperazone. In the present invention, cefoperazone, cefpiramide, cefatrizine, cephapirin, cefotiam, flomoxef, and cefaclor are preferable, and cefatrizine, cephapirin, cefotiam, cefoperazone, and cefpiramide are especially preferable.

The carbapenem series compounds used in the present invention are antibiotics belonging to such antibiotic series which, in combination with the penicillin series compounds or cephalosporin series compounds or pharmaceutically acceptable salts thereof, provide a synergistic antimicrobial activity. Such antibiotics are known carbopenem series compounds, namely imipenem, i.e., (5R,6S,8R)-3-[{2-(formimidoylamino)ethyl}thio]-6-(1-hydroxyethyl)-7-oxo-1-azabicyclo[3.2.0]hept-2-ene-2-carboxylic acid monohydrate, SM-7338, i.e., (4R,5S,6S,8R,2'S,4'S)-3-[2-(dimethylaminocarbonyl)pyrrolidin-4-ylthio]-6-(1-hydroxyethyl)-4-methyl-7-oxo-1-azabicyclo[3.2.0]hept-2-ene-2-carboxylic acid; and RS-533, i.e., (5R,6S,8R,4'S)-3-[1-acetimidoylpyrrolidin-4-ylthio]-6-(1-hydroxyethyl)-7-oxo-1-azabicyclo[3,2,0]hept-2-ene-2-carboxylic acid. In the present invention, imipenem is especially preferable.

The pharmaceutically acceptable salts of the carbapenem series compounds, penicillin series compounds, and of the cephalosporin series compounds are conventional non-toxic salts or solvates of these compounds. As the salts, there can be mentioned metal salts such as alkaline metal or alkaline earth metal salts such as sodium, potassium, calcium, magnesium, aluminium salts; organic amine salts such as N,N'-dibenzylethylenediamine salt, procaine salt; inorganic acid salts such as hydrochloride salt, hydrobromide salt, nitrate, sulfate, perchloric acid salt; salts of organic acids such as acetate, lactate, propyonate, maleate, fumarate, malate, tartrate, citrate; sulfonates such as methanesulfonate, isethionate, p-toluenesulfonate; and salts of amino acids such as salts of glutamic acid, aspartic acid, lysine, and of arginine. In the present invention, metal salts, salts of inorganic acids and salts of organic acids are preferable. As solvates hydrate, ethanol solvate, aceton solvate, and propylene glycol solvate can be mentioned. In the present invention, hydrate and propylene glycol solvate are preferable.

Next, a process far the production of the present antimicrobial composition is described.

An antimicrobial composition comprises (1) a first ingredient, i.e., a compound selected from penicillin series compounds and cephalosporin series compounds, and pharmaceutically acceptable salts thereof, and (2) a second ingredient, i.e., a compound selected from carbapenem series compounds and pharmaceutically acceptable salts thereof. In accordance with an embodiment of the present antimicrobial composition, the first ingredient and the second ingredient are formulated in one composition, and in another embodiment, the first ingredient and the second ingredient are separately formulated in different compositions.

The ratio of the first ingredient to the second ingredient can be widely varied, and is usually 1:1 to 600:1, preferably 1:1 to 200:1.

Formulations of the present antimicrobial composition may be liquid formulations such as an injectable solution or suspension, syrup, emulsion; solid formulations such as tablets, capsules, and powders; and ointments and suppositories. These formulations may contain usual additives or excipients, such as an adjuvant, stabilizer, wetting agent, emulsifier, absorption promoter, and surfactant. The additives or excipients are injectable distilled water, Ringer's solution, glucose, sucrose syrup, gelatin, edible oil, coconut oil, ethylene glycol, sucrose, corn starch, magnesium stearate, and talc. It is generally known that a carbapenem compound is metabolized by a renal dipeptidase, dehydropeptidase (E.C.3.4.13.11). Accordingly, where carbapenem compounds such as imipenem, RS-533, which are metabolized by the dehydropeptidase, are used in the present antimicrobial composition, the composition can contain a dehydropeptidase inhibitor which selectively inhibits the metabolism, such as cilastatin, i.e., sodium 7-[[(R)-amino-2-carboxyethyl]-thio]-2-[(S)-(2,2-dimethylcyclopropanecarboxamido]-2-heptenoate. Alternatively, the dehydropeptidase inhibitor can be separately administered at the same time as administering the present composition.

Where the carbapenem compound used is not metabolized as described above, it is not necessary for the present composition to contain the inhibitor.

The present antimicrobial composition can be administered in the same manner as conventional β-lactam antibiotics, i.e., can be administered parenterally, orally or externally. Preferably, it is administered by injection. The injectable composition is an injectable liquid such as a solution or suspension comprising the antibiotics and an injectable liquid medium as described above. Alternatively, the present composition can be solid composition which is dissolved in a suitable vehicle, such as sterile distilled water, physiological saline, immediately before use.

The present antimicrobial composition can be used to treat bacterial infectious diseases by pathogens which are gram-positive bacteria including methicillin resistant Staphylococcus aureus and methicillin resistant Staphylococcus epidermidis. In one embodiment, an antimicrobial composition comprising both

5

the first ingredient and the second ingredient is administered, and in another embodiment, the first ingredient and the second ingredient are separately administered at the same time or at different times through the same route or different routes. Where an antimicrobial composition comprising both the first ingredient and the second ingredient is administered, the total amount administered is generally 5 to 200 mg/kg/day, preferably 10 to 100 mg/kg/day. Where the first ingredient and the second ingredient are separately administered, the daily dose of the first ingredient is 5 to 200 mg/kg/day, preferably 10 to 100 mg/kg/day, and the daily dose of the second ingredient is 0.1 to 100 mg/kg/day, preferably 0.1 to 50 mg/kg/day. If desired, the present antimicrobial composition can be administered in 2 to 5 portions per day.

Next, the effectiveness and usefulness of the present antimicrobial composition are explained in detail.

The synergism of the antimicrobial composition is demonstrated by conventional methods, such as the disk diffusion method, fractional inhibitory concentration (FIC) index, and fractional effective dose (FED) index. Note, the FIC index is an indication showing an enhancement of the antimicrobial activity of a combination of antibiotics, and the FED index is an indication showing an enhancement of the antimicrobial activity in an experimental infections.

(1) Evaluation of synergistic effects by disk method

Test method

A predetermined amount of MRSA is swabbed on a Mueller-Hinton Agar (Difco, USA) plate to prepare a test plate. A paper disk having a diameter of 8 mm and containing 6 $\mu$g of a carbapenem series compound or a pharmaceutically acceptable salt thereof (abbreviated as "carbapenem" hereinafter), and a paper disk having a diameter of 8 mm and containing 100 $\mu$g of a compound selected from penicillin series compound and cephalosporin series compounds and pharmaceutically acceptable salts thereof (abbreviated as "partner antimicrobial compound" hereinafter) are positioned on the test plate at a distance of 17 mm between the paper disks. The test plate is incubated at 30°C for 18 hours, and an inhibitory zone formed around the disk containing the partner antimicrobial compound is measured. Where a synergism between carbapenem and the partner antimicrobial compound occurs, usually the inhibitory zone around the disk containing the partner antimicrobial compound expands toward the disc containing carbapenem, in comparison with the opposite side which does not feel the effect of the carbapenem. Accordingly, a radius of the expanded inhibitory zone and that of the opposite zone are measured, and a ratio of the former to the latter is calculated. A combination of antibiotics which provides a ratio of over at least 1.0 is evaluated as synergistic.

Table 1 shows a result wherein the synergism against MRSA BB 5918 was tested by using imipenem, SM-7388, and RS-533 as the carbapenem; and penicillin G, ampicillin, carbenicillin, piperacillin, cephaloridine, cefazolin, cephapirin, cefamandole, cefotiam, cefaclor, cefadroxil, cefuzoname, cefoperazone, cefpimizole, cefpiramide, and cefoxitin as the partner antimicrobial compound.

Table 1

| Synergism against MRSA BB 5918 of carbapenems and partner antimicrobial compounds (Ratio of radius of expanded inhibitory zone to that of opposite zone) | | | |
|---|---|---|---|
| Partner antimicrobial compound | Carbapenem | | |
| | Imipenem | SM-7338 | RS-533 |
| Penicillin G | 1.2 | 1.2 | 1.2 |
| Ampicillin | 1.2 | 1.2 | 1.2 |
| Carbenicillln | 1.1 | 1.0 | 1.0 |
| Piperacillin | 1.5 | 1.4 | 1.5 |
| Cephaloridine | 1.2 | 1.2 | 0.6 |
| Cefazolin | 1.5 | 1.3 | 1.5 |
| Cephapirin | 1.3 | 1.3 | 1.3 |
| Cefamandole | 1.2 | 1.7 | 1.2 |
| Cefotiam | 1.6 | 1.6 | 1.5 |
| Cefaclor | 1.4 | 1.3 | 1.5 |
| Cefadroxil | 1.3 | 1.0 | 1.0 |
| Cefuzoname | 1.6 | 1.0 | 1.4 |
| Cefoperazone | 2.1 | 2.3 | 2.1 |
| Cefpimizole | 3.6 | 1.3 | 2.7 |
| Cefpiramide | 1.4 | 1.4 | 1.5 |
| Cefoxitin | 1.1 | 1.0 | 1.1 |

Result

As seen from Table 1, combinations of the carbapenems and a partner antimicrobial compound selected from penicillin G, ampicillin, piperacillin cefazolin, cephapirin, cefamandole, cefotiam, cefaclor, cefoperazone, cefpimizole and cefpiramide provided a synergistic antimicrobial activity against MRSA BB 5918.

(2) Evaluation of synergistic effects by FIC index

Test method

Mueller-Hinton Agar plates containing a carbapenem compound alone, a partner antimicrobial compound alone, or a combination of the carbapenem compound and partner antimicrobial compound in a ratio by weight = 1:1012 to 1012:1 are prepared as checker board, about 5 $\mu$l of a MRSA cell suspension having a concentration of $10^6$ cells/ml is spotted on the agar plate, and after incubation at 30°C for 18 hours, the minimum inhibitory concentration (MIC) are measured.

Calculation of FIC index

The FIC index is calculated according to the following equation:

$$\text{FIC index} = \frac{C}{A} + \frac{D}{B}$$

wherein
   A:    MIC of a carbapenem alone;
   B:    MIC of a partner antimicrobial compound alone;
   C:    MIC of the carbapenem when used with the partner antimicrobial compound; and
   D:    MIC of the partner antimicrobial compound when used with the carbapenem.
   It is considered that where the FIC index $\leqq$ 0.5, the compound is synergistic; and where 0.5 < FIC index $\leqq$ 1.0 stands, the compound is partially synergistic.

7

Results

Table 2 shows the results of combinations of SM-7338 and cefotiam hydrochloride, imipenem and cefotiam hydrochloride, imipenem and cefpiramide sodium, imipenem and piperacillin sodium, imipenem and cefatrizine propylene glycol, imipenem and cefoperazone sodium, and imipenem and cephapirin sodium, against 25 strains of MRSA.

Table 2

Synergism of antimicrobial activity of carbapenems in
combination with partner antimicrobial compounds on
25 strains of MRSA

| Antibiotic | Range of MIC (μg/ml) | Geometric mean of MIC (μg/ml) | Arithmetic mean of FIC index |
|---|---|---|---|
| SM-7338 | 25 - 50 | 36.8 | |
| Cefotiam | 100 - 400 | 223.4 | 0.155 |
| SM-7338 (in combination) | 0.39 - 3.13 | 0.80 | |
| Cefotiam (in combination) | 12.5 - 50 | 25 | |
| Imipenem | 12.5 - 50 | 25 | |
| Cefotiam | 50 - 400 | 102 | 0.164 |
| Imipenem (in combination) | 0.025 - 0.2 | 0.062 | |
| Cefotiam (in combination) | 6.25 - 50 | 14.4 | |
| Imipenem | 6.25 - 50 | 18.9 | |
| Cefpiramide | 12.5 - 100 | 32.1 | 0.237 |
| Imipenem (in combination) | 0.012 - 1.56 | 0.064 | |
| Cefpiramide (in combination) | 3.13 - 12.5 | 6.98 | |
| Imipenem | 25 - 50 | 30.4 | |
| Piperacillin | 50 - 200 | 128.3 | 0.371 |
| Imienem (in combination) | 0.05 - 12.5 | 1.16 | |
| Piperacillin (in combination) | 0.39 - 50 | 27.9 | |

Table 2 (Continued)

| Antibiotic | Range of MIC ($\mu$g/ml) | Geometric mean of MIC ($\mu$g/ml) | Arithmetic mean of FIC index |
|---|---|---|---|
| Imipenem | 12.5 - 50 | 24.3 | |
| Cefatrizine | 12.5 - 50 | 28.7 | 0.329 |
| Imipenem (in combination) | 0.05 - 6.25 | 1.22 | |
| Cefatrizine (in combination) | 3.13 - 12.5 | 6.98 | |
| Imipenem | 6.25 - 50 | 25.7 | |
| Cefoperazone | 100 - 800 | 320 | 0.051 |
| Imipenem (in combination) | 0.025 - 0.2 | 0.054 | |
| Cefoperazone (in combination) | 6.25 - 25 | 12.8 | |
| Imipenem | 12.5 - 50 | 28.7 | |
| Cephapirin | 25 - 50 | 30.3 | 0.377 |
| Imipenem (in combination) | 0.05 - 12.5 | 0.551 | |
| Cephapirin (in combination) | 6.25 - 12.5 | 8.47 | |

In the test using 25 MRSA strains, all having an MIC of more than 400 $\mu$g/ml for methicillin, the combinations of SM-7338 and cefotiam, imipenem and cefotiam, and of imipenem and cefoperazone, provide a synergistic antimicrobial activity against all of the 25 MRSA strains tested, and the combination of imipenem and cefpiramide provided synergistic antimicrobial action against 24 of the 25 MRSA strains tested. Moreover, as seen from Table 2, all of the other combinations provided a synergistic antimicrobial activity against some of the strains tested.

(3) Evaluation of synergistic effects by FED index

Test method

Imipenem admixed with cilastatin sodium, a partner antimicrobial compound, and combinations of the imipenem and the partner antimicrobial compound in a predetermined ratio are dissolved in injectable sterile distilled water to prepare injectable antimicrobial compositions. A predetermined amount of MRSA is suspended in 5% mucin solution, and 0.5 ml of the suspension is intraperitoneally inoculated to ICR male mice (4 weeks old, body weight 19 to 21 g, n = 10). One hour after the injection, 0.2 ml of the injectable antimicrobial composition is subcutaneously injected, and 5 days later, the number of survival mice is counted to calculate the 50% effective dose.

Calculation of FED index

The FED index is calculated according to the following equation:

FED index $= \dfrac{c}{a} + \dfrac{d}{b}$

wherein

    a:     $ED_{50}$ value of imipenem;

    b:     $ED_{50}$ value of a partner antimicrobial compound;

    c:     $ED_{50}$ of imipenem when used with the partner antimicrobial compound; and

    d:     $ED_{50}$ of the partner antimicrobial compound when used with imipenem.

    Usually, a combination providing an FED index $\leq 0.5$ is considered to be synergistic.

Result

Tables 3-1 and 3-2 show the synergism of combinations of imipenem (admixed with cilastatin sodium (as cilastatin) in a ratio by weight of 1:1) and cefotiam in a ratio by weight of 1:5 to 1:600 against an infection by MRSA BB 5918 ($2.7 \times 10^8$ CFU/mouse) and MRSA pMS 520/Smith strain ($1.4 \times 10^7$ CFU/mouse), in mice.

Table 3-1

| Synergism of imipenem and cefotiam against the infection by MRSA BB 5918 strain | | | |
|---|---|---|---|
| Antibiotic | Ratio of imipenem/cefotiam | $ED_{50}$ (mg/kg) (95% confidence) | FED index |
| Imipenem* (alone)<br>Cefotiam (alone) | - | 50.0(28.1-89.4)<br>260(130-595) | - |
| Imipenem* (combination)<br>Cefotiam (combination) | 1/5 | 6.44(3.14-12.5)<br>32.0(15.5-62.3) | 0.252 |
| Imipenem* (combination)<br>Cefotiam (combination) | 1/10 | 5.04(2.20-9.74)<br>50.4(22.0-97.4) | 0.295 |
| Imipenem* (combination)<br>Cefotiam (combination) | 1/20 | 2.30(1.01-4.39)<br>45.5(20.0-86.8) | 0.221 |
| Imipenem* (combination)<br>Cefotiam (combination) | 1/40 | 1.37(0.59-2.71)<br>54.5(23.3-108) | 0.237 |
| Imipenem* (combination)<br>Cefotiam (combination) | 1/80 | 0.32(0.05-0.72)<br>25.8(4.40-58.0) | 0.105 |
| Imipenem* (combination)<br>Cefotiam (combination) | 1/160 | 0.22(0.07-0.43)<br>34.6(11.6-69.3) | 0.137 |

Imipenem* notes a mixture of imipenem and cilastatin sodium (as cilastatin) in a ratio by weight of 1:1.

Table 3-2

| Synergism of imipenem and cefotiam against the infection by MRSA pMS 520/Smith | | | |
|---|---|---|---|
| Antibiotic | Ratio of imipenem/ce fotiam | $ED_{50}$ (mg/kg) (95% confidence) | FED index |
| Imipenem* (alone) <br> Cefotiam (alone) | - | 6.73(2.62-13.1) <br> 61.4(27.5-124) | - |
| Imipenem* (combination) <br> Cefotiam (combination) | 1/6 | 0.89(0.08-2.04) <br> 5.31(0.48-12.2) | 0.219 |
| Imipenem* (combination) <br> Cefotiam (combination) | 1/20 | 0.34(0.09-0.63) <br> 6.65(1.77-12.2) | 0.159 |
| Imipenem* (combination) <br> Cefotiam (combination) | 1/60 | <0.21 <br> <12.3 | <0.231 |
| Imipenem* (combination) <br> Cefotiam (combination) | 1/200 | 0.053(0.021-0.10) <br> 10.5(4.22-19.6) | 0.179 |
| Imipenem* (combination) <br> Cefotiam (combination) | 1/600 | 0.013(0.003-0.028) <br> 7.76(1.54-16.6) | 0.128 |

Imipenem* notes a mixture of imipenem and cilastatin sodium (as cilastatin) in a ratio by weight of 1:1.

As seen from Tables 3-1 and 3-2, all of the combinations of imipenem and cefotiam in different ratios provided a remarkable synergism against the infection by MRSA BB 5918 and MRSA pMS 520/Smith, in mice.

Clinical Test 1.

A solution of 0.5 g (potency) of imipenem/0.5 g of cilastatin sodium (as cilastatin) and 2 g of cefotiam hydrochloride in physiological saline was intravenously injected to a male patient (age: 65 years; body weight: 62 kg) suffering from acute pneumonia, twice a day by 50 minutes in drip infusion.

Prior to starting this combination therapy, although the patient had intravenously received 2 g/day sodium flomoxef for 4 days, 2 g/day fosfomycin for 5 days, and 1.0 g (potency) imipenem/1.0 g cilastatin sodium (as cilastatin) for 4 days, the therapeutic effects were not satisfactory.

One day from the start of the combination therapy, the MRSA disappeared. Prior to the start of the combination therapy, a large amount of light brown sputum was observed, and the number of white blood cells (WBC) was 12400/$\mu$l. On the fourth day from start of the combination therapy the sputum decreased, on the sixth day the WBC was normal at 5300/$\mu$l, on the eighth day the sputum became transparent, and on the eleventh day the test was terminated. Side effects were not observed during the test.

Evaluation of synergistic effects by FIC idex

The synergism of the combination of imipenem and cefotiam on MRSA isolated from the sputum is shown in Table 4.

# EP 0 384 410 B1

Table 4

| Day | Administration to patient | In vitro test | | |
|---|---|---|---|---|
| | | Antibiotics | MIC (μg/ml) | FIC index |
| 4 days before start of test | Imipenem* (alone) | Imipenem (alone)<br>Cefotiam (alone)<br>Imipenem (combination)<br>Cefotiam (combination) | 50<br>200<br>0.1<br>25 | 0.127 |
| 1 day after start of test | Imipenem* and cefotiam (combination) | MRSA not detected | | |

Imipenem* notes a mixture of imipenem and cilastatin sodium (as cilastatin) in a ratio by weight of 1:1.

As seen from Table 4, the combination of imipenem and cefotiam provided a remarkable synergistic action against the MRSA infection.

Clinical experment 2.

A solution of 0.5 g (potency) of imipenem/0.5 g of cilastatin sodium (as cilastatin) and 2 g of cefotiam hydrochloride in physiological saline was intravenously injected to a female patient (age: 83 years; body weight: 37.3 kg) infected by MRSA, twice a day, by 30 minutes in drip infusion.

Just before the present combination therapy, although the patient had orally received 600 mg/day ofloxacin for 35 days, a therapeutic effect was not observed.

Although three days before the start of combination therapy the sputum containing a substantial amount of MRSA was observed, after an intravenous injection of 0.5 g (potency) of imipenem/0.5 g of cilastatin sodium (as cilastatin) twice a day by 30 minutes drip infusion, on the day of the start of the combination therapy, sputum containing MRSA was observed. Although on the third day of the combination therapy, a small amount of MRSA was detected, three days from the termination of the combination therapy on the 16th day, the MRSA disappeared. During the therapy, the body temperature lowered from about 38°C to about 37°C, and the inflammation was remarkably alleviated. The clinical symptom and the laboratory findings were remarkably improved, and side effects were not observed.

Evaluation of synergistic effects by FIC index

Similar to the clinical test 1, the synergism of the combination of imipenem and cefotiam against MRSA isolated from the sputum is shown in Table 5.

12

Table 5

| Day | Administration to patient | In vitro test | | |
|---|---|---|---|---|
| | | Antibiotic | MIC ($\mu$g/ml) | FIC index |
| 4 days before start of test | Imipenem* (alone) | Imipenem (alone) | 100 | |
| | | Cefotiam (alone) | 800 | 0.129 |
| | | Imipenem (combination) | 0.39 | |
| | | Cefotiam (combination) | 100 | |
| One day before start of test | Imipenem* (alone) | Imipenem (alone) | 25 | |
| | | Cefotiam (alone) | 6.25 | 0.502 |
| | | Imipenem (combination) | 0.025 | |
| | | Cefotiam (combination) | 3.13 | |
| Day of start of test | Imipenem* and cefotiam (combination) | Imipenem (alone) | 100 | |
| | | Cefotiam (alone) | 800 | 0.070 |
| | | Imipenem (combination) | 0.78 | |
| | | Cefotiam (combination) | 50 | |
| 4th day of combina- tion therapy | Imipenem* and cefotiam (combination) | Imipenem (alone) | 100 | |
| | | Cefotiam (alone) | 800 | 0.070 |
| | | Imipenem (combination) | 0.78 | |
| | | Cefotiam (combination) | 50 | |
| 8th day of combina- tion therapy | Imipenem* and cefotiam (combination) | Imipenem (alone) | 100 | |
| | | Cefotiam (alone) | 800 | 0.375 |
| | | Imipenem (combination) | 12.5 | |
| | | Cefotiam (combination) | 200 | |

Table 5 (continued)

| | | In vitro text | | |
|---|---|---|---|---|
| Day | Administration to patient | Antibiotic | MIC ($\mu$g/ml) | FIC index |
| 11th day of combina-tion therapy | Imipenem* and cefotiam (combination) | Imipenem (alone) | 50 | |
| | | Cefotiam (alone) | 400 | 0.502 |
| | | Imipenem (combination) | 0.1 | |
| | | Cefotiam (combination) | 200 | |
| 12th day of combina-tion therapy | Imipenem* and cefotiam (combination) | Imipenem (alone) | 50 | |
| | | Cefotiam (alone) | 400 | 0.504 |
| | | Imipenem (combination) | 0.2 | |
| | | Cefotiam (combination) | 200 | |
| 2 days after termina-tion of test | | MRSA not detected | | |

Imipenem* notes a mixture of imipenem and cilastetin sodium (as cilastatin) in a ratio by weight of 1:1.

As seen from Table 5, the combination of imipenem and cefotiam provided a remarkable synergism against clinically isolated MRSA, suggesting that an effective therapeutic action thereof exists against MRSA infections.

Formulation Example 1.

An aseptic mixture of cefotiam hydrochloride 475 mg, imipenem 25 mg, and cilastatin sodium 25 mg is filled and sealed in a sterilized vial. Upon use, the mixture is dissolved in physiological saline to prepare an injectable solution.

Formulation Example 2.

An aseptic mixture of cefpiramide sodium 400 mg, imipenem 80 mg, and cilastatin sodium 80 mg is dissolved in 20 ml of physiological saline, the solution is filtered through a 0.22 $\mu$m Millipore filter, and filled and sealed in a glass bottle, which has been sterilized, to prepare an injectable solution.

Formulation Example 3.

An aseptic mixture of cephapirin sodium 500 mg, imipenem 50 mg, and cilastatin sodium 50 mg is filled and sealed in a sterilized vial. Upon use, the mixture is dissolved in physiological saline to prepare an

14

injectable solution.

Formulation Example 4.

An aseptic mixture of ampicillin sodium 225 mg, imipenem 25 mg, and cilastatin sodium 25 mg is dissolved in 20 ml of physiological saline, the solution is filtered through a 0.22 $\mu$m Millipore filter, and filled and sealed in a glass bottle, which has been sterilized, to prepare an injectable solution.

Formulation Example 5.

An aseptic mixture of cefoperazone sodium 900 mg, imipenem 100 mg and cilastatin sodium 100 mg is filled and sealed in a sterilized vial. Upon use, the mixture is dissolved in physiological saline to prepare an injectable solution.

Formulation Example 6.

An aseptic mixture of cefotiam hydrochloride 475 mg, and SM-7338 25 mg is filled and sealed in a sterilized vial. Upon use, the mixture is dissolved in physiological saline to prepare an injectable solution.

Formulation Example 7.

An aseptic mixture of cefpiramide sodium 400 mg, and SM-7338 80 mg is dissolved in 20 ml of physiological saline, the solution is filtered through a 0.22 $\mu$m Millipore filter, and filled and sealed in a glass bottle, which has been sterilized, to prepare an injectable solution.

Formulation Example 8.

An aseptic mixture of cephapirin sodium 500 mg, SM-7338 50 mg and cilastatin sodium 50 mg is filled and sealed in a sterilized vial. Upon use, the mixture is dissolved in physiological saline to prepare an injectable solution.

Formulation Example 9.

An aseptic mixture of cefpiramide sodium 400 mg, RS-533 80 mg and cilastatin sodium 80 mg is dissolved in 20 ml of physiological saline, the solution is filtered through a 0.22 $\mu$m Millipore filter, and filled and sealed in a glass bottle, which has been sterilized, to prepare an injectable solution.

Formulation Example 10.

An aseptic mixture of cephapirin sodium 500 mg, RS-533 50 mg and cilastatin sodium 50 mg is filled and sealed in a sterilized vial. Upon use, the mixture is dissolved in physiological saline to prepare an injectable solution.

Formulation Example 11.

An aseptic mixture of ampicillin sodium 225 mg, RS-533 25 mg and cilastatin sodium 25 mg is dissolved in 20 ml of physiological saline, the solution is filtered through a 0.22 $\mu$m Millipore filter, and filled and sealed in a glass bottle, which has been sterilized, to prepare an injectable solution.

Formulation Example 12.

An aseptic mixture of cefoperazone sodium 900 mg, RS-533 100 mg and cilastatin sodium 100 mg is filled and sealed in a sterilized vial. Upon use, the mixture is dissolved in physiological saline to prepare an injectable solution.

The antimicrobial composition of the present invention provides an excellent antimicrobial activity against MRSA, which is a pathogen of intractable diseases, and therefore, the present invention provides an antimicrobial composition effective against pathogens which are highly resistant to $\beta$-lactam antibiotics.

15

**Claims**

1. An antimicrobial composition comprising a combination of (1) a first ingredient selected from penicillin series compounds, cephalosporin series compounds and pharmaceutically acceptable salts thereof; and (2) a second ingredient selected from carbapenem series compounds and pharmaceutically acceptable salts thereof wherein the penicillin series compound is selected from penicillin G, ampicillin, amoxicillin, mecillinam, pivmecillinam, carbenicillin, carfecillin, carindacillin, sulbenicillin, talampicillin, bacampicillin, ticarcillin, piperacillin, ciclacillin and hetacillin, the cephalosporin series compound is selected from cefatrizine, cefamandole, cefuzoname, cefpimizole, cephapirin, cephaloridine, cefsulodin, cefotiam, ceforanide, ceftexzole, cefoxitin, latamoxef, flomoxef, cefmetazole, cefotetan, cefpiramide, cephaloglycin, cephalexin, cefadroxil, cefroxadine, ceferadine, cefaclor and cefoperazone, the carbapenem series compound is selected from imipenem, i.e., (5R,6S,8R)-3-[{2-(formimidoylamino)-ethyl}thio] -6-(1-hydroxyethyl)-7-oxo-1-azabicyclo[3,2,0]hept-2-ene-2-carboxylic acid monohydrate, SM-7338, i.e. (4R,5S,6S,8R,2'S,4'S)-3-[2-(dimethylaminocarbonyl)-pyrrolidin-4-ylthio]-6-(1-hydroxy-ethyl)-4-methyl-7-oxo-1-azabicyclo[3,2,0]hept-2-ene-2-carboxylic acid, and RS-533,i.e. (5R,6S, 8R,4'S)-3-[1-acetimidoylpyrrolidin-4-ylthio]-6-(1-hydroxyethyl)-7-oxo-1-azabicyclo[3,2,0]-hept-2-ene-2-carboxylic acid; and said combination providing a synergistic effect over the compounds used alone.

2. An antimicrobial composition according to claim 1 comprising a combination of (1) a first ingredient selected from the penicillin series compounds and pharmaceutically acceptable salts thereof; and (2) a second ingredient selected from the carbapenem series compounds and pharmaceutically acceptable salts thereof.

3. An antimicrobial composition according to claim 1 comprising a combination of (1) a first ingredient selected from the cephalosporin series compounds and pharmaceutically acceptable salts thereof; and (2) a second ingredient selected from the carbapenem series compounds and pharmaceutically acceptable salts thereof.

4. An antimicrobial composition according to claim 2, wherein the penicillin series compound is selected from ampicillin and piperacillin.

5. An antimicrobial composition according to claim 4, wherein the penicillin series compound is piperacillin.

6. An antimicrobial composition according to claim 3, wherein the cephalosporin series compound is selected from cefoperazone, cefpiramide, cefatrizine, cefotiam, cephapirin, flomoxef and cefaclor.

7. An antimicrobial composition according to claim 6, wherein the cephalosporin series compound is selected from cefoperazone, cefpiramide, cefatrizine, cephapirin and cefotiam.

8. An antimicrobial composition according to claim 1, wherein the carbapenem series compound is imipenem.

9. An antimicrobial composition according to claim 1, wherein a ratio by weight of the first ingredient to the second ingredient is 1:1 to 600:1.

10. An antimicrobial composition according to claim 9, wherein the ratio by weight of the first ingredient to the second ingredient is 1:1 to 200:1.

11. The use of an antimicrobial composition comprising a combination of (1) a first ingredient selected from penicillin series compounds, cephalosporin series compounds and pharmaceutically acceptable salts thereof; and (2) a second ingredient selected from carbapenem series compounds and pharmaceutically acceptable salts thereof wherein the penicillin series compound is selected from penicillin G, ampicillin, amoxicillin, mecillinam, pivmecillinam, carbenicillin, carfecillin, carindacillin, sulbenicillin, talampicillin, bacampicillin, ticarcillin, piperacillin, ciclacillin and hetacillin, the cephalosporin series compound is selected from cefatrizine, cefamandole, cefuzoname, cefpimizole, cephapirin, cephaloridine, cefsulodin, cefotiam, ceforanide, ceftexzole, cefoxitin, latamoxef, flomoxef, cefmetazole, cefotetan, cefpiramide, cephaloglycin, cephalexin, cefadroxil, cefroxadine, ceferadine, cefaclor and cefoperazone, the carbapenem series compound is selected from imipenem, i.e., (5R,6S,8R)-3-[{2-(formimidoylamino)ethyl}thio]-6-(1-hydroxyethyl)-7-oxo-1-azabicyclo[3,2,0]hept-2-ene-2-carboxylic acid monohydrate, SM-7338, i.e.; (4R,5S,6S,8R,2'S, 4'S)-3-[2-(dimethylaminocarbonyl)pyrrolidin-4-ylthio]-6-(1-hydroxy-ethyl)-4-methyl-7-oxo-1-azabicyclo[3,2,0] hept-2-ene-2-carboxylic acid, and RS-533, i.e. (5R,6S,8R,4'S)-3-[1-acetimidoylpyrrolidin-4-ylthio] -6-(1-hydroxyethyl)-7-oxo-1-azabicyclo[3,2,0]-hept-2-ene-2-carboxylic acid; and said combination providing a synergistic effect over the compounds used alone for producing a medicament for treating MRSA infections.

**12.** Products containing a first antibiotic and a second antibiotic, wherein the first antibiotic is selected from penicillin series compounds, cephalosporin series compounds and pharmaceutically acceptable salts thereof and the second antibiotic is selected from carbapenem series compounds and pharmaceutically acceptable salts thereof, and wherein the penicillin series compound is selected from penicillin G, ampicillin, amoxicillin, mecillinam, pivmecillinam, carbenicillin, carfecillin, carindacillin, sulbenicillin, talampicillin, bacampicillin, ticarcillin, piperacillin, ciclacillin and hetacillin, the cephalosporin series compound is selected from cefatrizine, cefamandole, cefuzoname, cefpimizole, cephapirin, cephaloridine, cefsulodin, cefotiam, ceforanide, ceftexzole, cefoxitin, latamoxef, flomoxef, cefmetazole, cefotetan, cefpiramide, cephaloglycin, cephalexin, cefadroxil, cefroxadine, ceferadine, cefaclor and cefoperazone, the carbapenem series compound is selected from imipenem, i.e., (5R,6S,8R)-3-[{2-(formimidoylamino)ethyl}thio] -6-(1-hydroxyethyl)-7-oxo-1-azabicyclo[3,2,0]hept-2-ene-2-carboxylic acid monohydrate, SM-7338, i.e. (4R,5S,6S,8R,2'S,4'S)-3-[2-(dimethylaminocarbonyl)-pyrrolidin-4-ylthio]-6-(1-hydroxy-ethyl)-4-methyl-7-oxo-1-azabicyclo 3,2,0]hept-2-ene-2-carboxylic acid, and RS-533,i.e. (5R,6S, 8R,4'S)-3-[1-acetimidoylpyrrolidin-4-ylthio]-6-(1-hydroxyethyl)-7-oxo-1-azabicyclo[3,2,0]-hept-2-ene-2-carboxylic acid; and said combination providing a synergistic effect over the compounds used alone, the product containing the first antibiotic and the second antibiotic as a combined preparation for separate use or separately administering in the MRSA infection therapy.

**Patentansprüche**

**1.** Eine antimikrobielle Zusammensetzung, umfassend eine Kombination aus (1) einem ersten Bestandteil, ausgewählt aus Verbin dungen der Penicillinreihe, Verbindungen der Cephalosporinreihe und pharmazeutisch annehmbaren Salzen davon und aus (2) einem zweiten Bestandteil, ausgewählt aus Verbindungen der Carbapenemreihe und pharmazeutisch annehmbaren Salzen davon, wobei die Verbindung der Penicillinreihe aus Penicillin G, Ampicillin, Amoxicillin, Mecillinam, Pivmecillinam, Carbenicillin, Carfecillin, Carindacillin, Sulbenicillin, Talampicillin, Bacampicillin, Ticarcillin, Piperacillin, Ciclacillin und Hetacillin ausgewählt ist, die Verbindung der Cephalosporinreihe aus Cefatrizin, Cefamandol, Cefuzonam, Cefpimizol, Cephapirin, Cephaloridin, Cefsulodin, Cefotiam, Ceforanid, Ceftexzol, Cefoxitin, Latamoxef, Flomoxef, Cefmetazol, Cefotetan, Cefpiramid, Cephaloglycin, Cephalexin, Cefadroxil, Cefroxadin, Ceferadin, Cefaclor und Cefoperazon ausgewählt ist, die Verbindung der Carbapenemreihe aus Imipenem, d.h. (5R,6S,8R)-3-[{2-(Formimidoylamino)ethyl} thio]-6-(1-hydroxyethyl)-7-oxo-1-azabicyclo[3,2,0]-hept-2-en-2-carbonsäuremonohydrat, SM-7338, d.h. (4R,5S,6S,8R,2'S,4'S)-3-[2-(Dimethylaminocarbonyl)-pyrrolidin-4-ylthio]-6-(1-hydroxyethyl)-4-methyl-7-oxo-1-azabicyclo[3,2,0]hept-2-en-2-carbonsäure, und RS-533, d.h. (5R,6S,8R,4'S)-3-[1-Acetimidoylpyrrolidin-4-ylthio]-6-(1-hydroxyethyl)-7-oxo-1-azabicyclo[3,2,0]-hept-2-en-2-carbonsäure ausgewählt ist, wobei die Kombination einen synergistischen Effekt über die einzeln verwendeten Verbindungen bewirkt.

**2.** Antimikrobielle Zusammensetzung nach Anspruch 1, umfassend eine Kombination aus (1) einem ersten Bestandteil, ausgewählt aus den Verbindungen der Penicillinreihe und pharmazeutisch annehmbaren Salzen davon und aus (2) einem zweiten Bestandteil, ausgewählt aus den Verbindungen der Carbapenemreihe und pharmazeutisch annehmbaren Salzen davon.

**3.** Antimikrobielle Zusammensetzung nach Anspruch 1, umfassend eine Kombination aus (1) einem ersten Bestandteil, ausgewählt aus den Verbindungen der Cephalosporinreihe und pharmazeutisch annehmbaren Salzen davon und aus (2) einem zweiten Bestandteil ausgewählt aus den Verbindungen der Carbapenemreihe und pharmazeutisch annehmbaren Salzen davon.

**4.** Antimikrobielle Zusammensetzung nach Anspruch 2, worin die Verbindung der Penicillinreihe aus Ampicillin und Piperacillin ausgewählt ist.

**5.** Antimikrobielle Zusammensetzung nach Anspruch 4, worin die Verbindung der Penicillinreihe Piperacillin ist.

**6.** Antimikrobielle Zusammensetzung nach Anspruch 3, worin die Verbindung der Cephalosporinreihe aus Cefoperazon, Cefpiramid, Cefatrizin, Cefotiam, Cephapirin, Flomoxef und Cefaclor ausgewählt ist.

**7.** Antimikrobielle Zusammensetzung nach Anspruch 6, worin die Verbindung der Cephalosporinreihe aus Cefoperazon, Cefpiramid, Cefatrizin, Cephapirin und Cefotiam ausgewählt ist.

**8.** Antimikrobielle Zusammensetzung nach Anspruch 1, worin die Verbindung der Carbapenemreihe Imipenem ist.

**9.** Antimikrobielle Zusammensetzung nach Anspruch 1, worin ein Gewichtsverhältnis von erstem Bestandteil zu zweitem Bestandteil 1:1 bis 600:1 beträgt.

**10.** Antimikrobielle Zusammensetzung nach Anspruch 9, worin das Gewichtsverhältnis von erstem Bestandteil zu zweitem Bestandteil 1:1 bis 200:1 beträgt.

**11.** Verwendung einer antimikrobiellen Zusammensetzung, umfassend eine Kombination aus (1) einem ersten Bestandteil ausgewählt aus Verbindungen der Penicillinreihe, Verbindungen der Cephalosporinreihe und pharmazeutisch annehmbaren Salzen davon, und aus (2) einem zweiten Bestandteil, ausgewählt aus Verbindungen der Carbapenemreihe und pharmazeutisch annehmbaren Salzen davon, worin die Verbindungen der Penicillinreihe aus Penicillin G, Ampicillin, Amoxicillin, Mecillinam, Pivmecillinam, Carbenicillin, Carfecillin, Carindacillin, Sulbenicillin, Talampicillin, Bacampicillin, Ticarcillin, Piperacillin, Ciclacillin und Hetacillin ausgewählt ist, die Verbindung der Cephalosporinreihe aus Cefatrizin, Cefamandol, Cefuzonam, Cefpimizol, Cephapirin, Cephaloridin, Cefsulodin, Cefotiam, Ceforanid, Ceftexzol, Cefoxitin, Latamoxef, Flomoxef, Cefmetazol, Cefotetan, Cefpiramid, Cephaloglycin, Cephalexin, Cefadroxil, Cefroxadin, Ceferadin, Cefaclor und Cefoperazon ausgewählt ist, die Verbindung der Carbapenemreihe aus Imipenem, d.h. (5R,6S,8R)-3-[{2-(Formimidoylamino)ethyl}thio]-6-(1-hydroxyethyl)-7-oxo-1-azabicyclo[3,2,0]hept-2-en-2-carbonsäuremonohydrat, SM-7338, d.h. (4R,5S,6S,8R,2'S,4'S)-3-[2-(Dimethylaminocarbonyl)-pyrrolidin-4-ylthio]-6-(1-hydroxyethyl)-4-methyl-7-oxo-1-azabicyclo[3,2,0]hept-2-en-2-carbonsäure, und RS-533, d.h. (5R,6S, 8R,4'S)-3-[1-Acetimidoylpyrrolidin-4-ylthio]-6-(1-hydroxyethyl)-7-oxo-1-azabicyclo[3,2,0]-hept-2-en-2-carbonsäureausgewähltist und wobei die Kombination eine synergistische Wirkung über die einzeln verwendeten Verbindungen liefert, zur Herstellung eines Medikamentes zur Behandlung von MRSA-Infektionen.

**12.** Produkte, die ein erstes Antibiotikum und ein zweites Antibiotikum enthalten, wobei das erste Antibiotikum aus Verbindungen der Penicillinreihe, Verbindungen der Cephalosporinreihe und pharmazeutisch annehmbaren Salzen davon ausgewählt ist und das zweite Antibiotikum aus Verbindungen der Carbapenemreihe und pharmazeutisch annehmbaren Salzen davon ausgewählt ist und worin die Verbindung der Penicillinreihe aus Penicillin G, Ampicillin, Amoxicillin, Mecillinam, Pivmecillinam, Carbenicillin, Carfecillin, Carindacillin, Sulbenicillin, Talampicillin, Bacam picillin, Ticarcillin, Piperacillin, Ciclacillin und Hetacillin ausgewählt ist, die Verbindung der Cephalosporinreihe aus Cefatrizin, Cefamandol, Cefuzonam, Cefpimizol, Cephapirin, Cephaloridin, Cefsulodin, Cefotiam, Ceforanid, Ceftexzol, Cefoxitin, Latamoxef, Flomoxef, Cefmetazol, Cefotetan, Cefpiramid, Cephaloglycin, Cephalexin, Cefadroxil, Cefroxadin, Ceferadin, Cefaclor und Cefoperazon ausgewählt ist, die Verbindung der Carbapenemreihe aus Imipenem, d.h. (5R,6S,8R)-3-[{2-(Formimidoylamino)ethyl}thio]-6-(1-hydroxyethyl)-7-oxo-1-azabicyclo[3,2,0]-hept-2-en-2-carbonsäuremonohydrat, SM-7338, d.h. (4R,5S,6S,8R,2'S,4'S)-3-[2-(Dimethylaminocarbonyl)-pyrrolidin-4-ylthio]-6-(1-hydroxyethyl)-4-methyl-7-oxo-1-azabicyclo[3,2,0]hept-2-en-2-carbonsäure, und RS-533, d.h. (5R,6S,8R, 4'S)-3-[1-Acetimidoylpyrrolidin-4-ylthio]-6-(1-hydroxyethyl)-7-oxo-1-azabicyclo[3,2,0]-hept-2-en-2-carbonsäure ausgewählt ist, wobei die Kombination eine synergistische Wirkung über die einzeln verwendeten Verbindungen liefert, und das Produkt das erste Antibiotikum und das zweite Antibiotikum als ein kombiniertes Präparat zur getrennten Behandlung oder getrennten Verabreichung bei der Therapie von MRSA-Infektion enthält.

**Revendications**

1. Une composition antimicrobienne comprenant une combinaison de
   (1) un premier ingrédient choisi parmi les composés des séries des pénicillines, et des céphalosporines et sels pharmaceutiquement acceptables en dérivant et
   (2) un deuxième ingrédient choisi parmi les composés de la série des carbapenem et des sels pharmaceutiques en dérivant,
   composition dans laquelle le composé de la série de la pénicilline est choisi dans le groupe comprenant pénicilline G, ampicilline, amoxicilline, mécilliname, pivmecilliname, carbénicilline, carfécilline, carindacilline, sulbénicilline, talampicilline, bacampicilline, ticarcilline, pipéracilline, ciclacilline et hétacilline, les composés de la série de la céphalosporine sont choisis dans le groupe comprenant céfatrizine, céfamandole, céfuzoname, cefpimizole, cephapirine, céphaloridine, cefsulodine, cefotiame, ceforanide, ceftexzole, cefoxitine, latamoxef, flomexef, cefmetazole, cefotétane, cefpiramide, cephaloglycine, cefalexine, cefadroxile, cefroxadine, ceféradine, cefaclor et céfopérazone, le composé de la série des carbapenems est choisi dans le groupe comprenant imipenem, c'est-à-dire monohydrate de l'acide (5R, 6S, 8R)-3-[{2-(formimidoylamino)éthyl}thio]-6-(1-hydroxyéthyl)-7-oxo-1-azabicyclo[3,2,0]-hept-2-ène-2-carboxylique, SM-7338, c'est-à-dire acide (4R,5S,6S,8R,2'S, 4'S)-3-[2-(diméthylaminocarbonyl)-pyrrolidine-4-ylthio]-6-(1-hydroxy-éthyl)-4-méthyl-7-oxo-1-azabicyclo[3,2,0]hept-2-ène-2-carboxylique, et RS-533, c'est-à-dire acide (5R, 6S,8R,4'S)-3-[1-acétimidoylpyrrolidin-4-ylthio]-6-(1-hydroxyéthyl)-7-oxo-1-azabicyclo[3,2,0)-hept-2-ène-2-carboxylique; ladite combinaison conduisant à un effet synergique par rapport à l'usage des composés seuls.

2. Le composé antimicrobien selon la revendication 1, comprenant la combinaison de
   (1) un premier ingrédient choisi dans le groupe consistant en les composés de la série des pénicillines et des sels pharmaceutiquement acceptables en dérivant et
   (2) un deuxième ingrédient choisi dans le groupe consistant en les composés de la série des carbapénems et des sels pharmaceutiquement en dérivant.

3. Une composition antimicrobienne selon la revendication 1, comprenant une combinaison de
   (1) un premier ingrédient choisi parmi les composés de la série des céphalosporines et des sels pharmaceutiquement acceptables en dérivant et
   (2) un deuxième ingrédient choisi parmi les composés de la série des carbapénems et des sels pharmaceutiquement acceptables en dérivant.

4. Une composition antimicrobienne selon la revendication 2, dans laquelle le composé de la série des pénicillines est choisi dans le groupe comprenant ampicilline et pipéracilline.

5. Une composition antimicrobienne selon la revendication 4, dans laquelle le composé de la série des pénicillines est la pipéracilline.

6. Une composition antimicrobienne selon la revendication 3, dans laquelle le composé de la série des céphalosporines est choisi dans un groupe comprenant céfopérazone, cefpiramide, cefatrizine, cefotiam, céphapirine, flomoxef et céfaclor.

7. Une composition antimicrobienne selon la revendication 6, dans laquelle le composé de la série des céphalosporines est choisi dans le groupe comprenant cefopérazone, cefpiramide, cefatrizine, cephapirine et cefotiam.

8. Une composition antimicrobienne selon la revendication 1, dans laquelle le composé de la série des carbapenems est l'imipenem.

9. Une composition antimicrobienne selon la revendication 1, dans laquelle le rapport en poids premier ingrédient/deuxième ingrédient est compris entre 1/1 à 600/1.

10. Une composition antimicrobienne selon la revendication 9, dans lequelle le rapport en poids premier ingrédient/deuxième ingrédient est compris entre 1/1 et 200/1.

**11.** L'utilisation d'une composition antimicrobienne comprenant une combinaison de

(1) un premier ingrédient choisi parmi les composés de la série des pénicillines, des céphalosporines et des sels pharmaceutiquement acceptables en dérivant; et

(2) un deuxième ingrédient choisi parmi composés de la série des carbapenems et des sels pharmaceutiquement acceptables en dérivant,

composition dans laquelle le composé de la série des pénicillines est choisi dans le groupe comprenant pénicilline G, ampicilline, amoxicilline, mécilliname, pivmecilliname, carbénicilline, carfécilline, carindacilline, sulbénicilline, talampicilline, bacampicilline, ticarcilline, pipéracilline, ciclacilline et hétacilline, les composés de la série des céphalosporines sont choisis dans le groupe comprenant céfatrizine, céfamandole, céfuzoname, cefpimizole, cephapirine, céphaloridine, cefsulodine, cefotiame, ceforanide, ceftexzole, cefoxitine, latamoxef, flomexef, cefmetazole, cefotétane, cefpiramide, cephaloglycine, cephalexine, cefadroxil, cefroxadine, ceféradine, cefaclor et céfopérazone, le composé de la série des carbapenems est choisi dans le groupe comprenant imipenem, c'est-à-dire monohydrate de l'acide (5R, 6S, 8R)-3-[{2-(formimidoylamino)éthyl}thio]-6-(1-hydroxyéthyl)-7-oxo-1-azabicyclo[3,2,0]-hept-2-ène-2-carboxylique, SM-7338, c'est-à-dire acide (4R,5S,6S,8R,2'S, 4'S)-3-[2-(diméthylaminocarbonyl) pyrrolidine-4-ylthio]-6-(1-hydroxy-éthyl)-4-méthyl-7-oxo-1-azabicyclo[3,2,0]hept-2-ène-2-carboxylique, et RS-533, c'est-à-dire acide (5R, 6S,8R,4'S)-3-[1-acétimidoylpyrrolidin-4-ylthio]-6-(1-hydroxyéthyl)-7-oxo-1-azabicyclo[3,2,0)-hept-2-ène-2-carboxylique; ladite combinaison conduisant à un effet synergique par rapport aux composés utilisés seuls, dans le but de produire un médicament destiné à traiter les infections MRSA.

**12.** Produits contenant un premier antibiotique et un deuxième antibiotique, dans lequel le premier antibiotique est choisi parmi les composés de la série des pénicillines, de la série des céphalosporines et des sels pharmaceutiquement acceptables en dérivant, et le deuxième antibiotique est choisi parmi les composés de la série des carbopenems et des sels pharmaceutiquement acceptables en dérivant, et dans laquelle le composé de la série des pénicillines est choisi dans le groupe comprenant pénicilline G, ampicilline, amoxicilline, mécilliname, pivmecilliname, carbénicilline, carfécilline, carindacilline, sulbénicilline, talampicil-line, bacampicilline, ticarcilline, pipéracilline, ciclacilline et hétacilline, les composés de la série des céphalosporines sont choisis dans le groupe comprenant céfatrizine, céfamandole, céfuzoname, cefpimizole, cephapirine, céphaloridine, cefsulodine, cefotiame, ceforanide, ceftexzole, cefoxitine, latamoxef, flomexef, cefmetazole, cefotétane, cefpiramide, cephaloglycine, cephalexine, cefadroxil, cefroxadine, ceféradine, cefaclor et céfopérazone, le composé de la série des carbapenem est choisi dans le groupe comprenant imipenem, c'est-à-dire monohydrate de l'acide (5R, 6S, 8R)-3-[{2-(formimidoylamino)éthyl}thio]-6-(1-hydroxyéthyl)-7-oxo-1-azabicyclo[3,2,0]hept-2-ène-2-carboxylique, SM-7338, c'est-à-dire acide (4R,5S,6S,8R,2'S, 4'S)-3-[2-(diméthylaminocarbonyl)-pyrrolidine-4-ylthio]-6-(1-hydroxy-éthyl)-4-méthyl-7-oxo-1-azabicyclo[3,2,0]hept-2-ène-2-carboxylique, et RS-533, c'est-à-dire acide (5R, 6S,8R,4'S)-3-[1-acétimidoylpyrrolidin-4-ylthio]-6-(1-hydroxyéthyl)-7-oxo-1-azabicyclo[3,2,0)-hept-2-ène-2-carboxylique; et ladite combinaison conduisant à un effet synergique par rapport aux composés utilisés seuls, le produit contenant le premier antibiotique et le second antibiotique en tant que préparation combinée pour un usage particulier ou une administration particulière, dans le cadre d'une thérapie de l'infection MRSA.